(19) Europäisches Patentamt
European Patent Office
Office européen des brevets

(11) **EP 2 198 780 B1**

(12) **EUROPEAN PATENT SPECIFICATION**

(45) Date of publication and mention of the grant of the patent:
**31.01.2018 Bulletin 2018/05**

(51) Int Cl.:
**G01B 11/25** *(2006.01)*      **A61B 5/103** *(2006.01)*
**G06T 7/55** *(2017.01)*

(21) Application number: **09180034.2**

(22) Date of filing: **18.12.2009**

(54) **Method and device for optical scanning of three-dimensional objects by means of a dental 3D camera using a triangulation method**

Verfahren und Vorrichtung zur optischen Vermessung von dreidimensionalen Objekten mittels einer dentalen 3D-Kamera unter Verwendung eines Triangulationsverfahrens

Dispositif et procédé de mesure optique d'objet tridimensionnel avec une caméra dentaire 3D utilisant le procédé de triangulation

(84) Designated Contracting States:
**AT CH DE DK FR GB IT LI**

(30) Priority: **19.12.2008 DE 102008054985**
**21.09.2009 US 563723**

(43) Date of publication of application:
**23.06.2010 Bulletin 2010/25**

(73) Proprietor: **Sirona Dental Systems GmbH**
**64625 Bensheim (DE)**

(72) Inventors:
• **Pfeiffer, Joachim**
**64625, Bensheim (DE)**

• **Klein, Konrad**
**69121 Heidelberg (DE)**

(74) Representative: **Sommer, Peter**
**PRIO Patentanwälte**
**Augustaanlage 22**
**68165 Mannheim (DE)**

(56) References cited:
**EP-A2- 0 250 993          JP-A- 2008 190 962**
**US-A1- 2006 192 082**

## Description

Technical field

The invention relates to a method and a device for optical scanning of three-dimensional objects by means of a dental 3D camera using a triangulation method. To this end, several individual images of a pattern projected on the object are recorded.

Prior art

With prior methods, the user has had to fix the moment of initiation by actuating a footswitch and has had to hold the camera still from this moment on for the duration of the exposure. Recording was carried out at the moment of initiation and the result then visualized. When assessing the resulting image, a certain degree of experience is required for recognition of artifacts caused by camera shake and, if necessary, for a decision to erase the recording. The actuation of the footswitch is likely to increase movement at the moment of initiation. The problem of camera shake was thus solved by making a plurality of recordings until one occurred in which, according to the judgment of the user, no artifacts caused by camera shake could be detected, this being kept for use whilst the other images were deleted.

The actuation of the footswitch is likely to increase movement at the moment of initiation.

This is solved in DE 10 2005 020 240 A1 by a delay circuit, which causes scanning to take place only after the elapse of an adjustable time interval following actuation of the initiator by the user. In this way, the user has the possibility of resetting the camera into a state of rest following actuation of the initiating means. However, this method also requires the experience of the user for assessment of camera shake in the individual recordings.

The attainment of low-shake recordings is, in fact, one of the main difficulties for the dentist when acquiring competence in the recording method making use of triangulation by means of a handheld camera. Hitherto, the problem has been solved by the experience of the user.

In order to avoid camera shake, the display of a video image as viewfinder image is not purposeful, since the artifacts caused by camera shake cannot be recognized in advance but are only visible after the user has initiated an exposure. This range of problems is described in EP 0,250,993 B1.

The acquisition of tooth geometries with a handheld 3D camera involves the problem of camera shake. Particularly in the case of the intra-oral situation, but also in many situations involving extra-oral scanning of plaster casts, this problem cannot be completely avoided by resting on, supporting or the like. Camera shake is particularly detrimental in the case of 3D recording using structurized light and the phase shifting method, since in this case a plurality of exposures, usually from 4 to 16 exposures, are required from the same camera position.

In a dental 3D camera disclosed in DE 198 29 278, the monomode range is increased by partially covering a diaphragm mechanically during a scanning sequence, so that scanning cannot be continuously repeated. The camera movements during the individual images as caused by camera shake lead here to wavy artifacts and sometimes completely useless 3D exposures.

Another three-dimensional measurement apparatus for reducing the effect due to hand movement is disclosed in JP 2008 190962 A.

Summary of the invention

It is an object of the invention to provide a method and device of the aforementioned type which avoids the said drawbacks and assists the user in obtaining almost non-shake images and, in particular, makes it possible to indicate in a suitable manner the occurrence of camera shake in real time and to automatically detect moments at which no camera shake or only slight camera shake of the 3D camera takes place or has taken place.

A method for optically scanning a threedimensional object is claimed in claim 1. In the claimed method, a dental 3D camera operates in accordance with a triangulation procedure to acquire a plurality of images of the object, the method including the following steps: forming at least one comparative signal based on at least two images of the object acquired by the camera while at least one pattern is projected on the object; and determining at least one camera shake index based on the at least one comparative signal.

This camera shake index provides an objective measure of the camera shake and can therefore be used for assessment of the quality of the optical scan.

A 3D data set can be made from a scanning sequence consisting of at least two images of the pattern projected on the object, and advantageously at least one of the images used for computation of the camera shake index forms part of the scanning sequence.

The use of images from the scanning sequence for computation of the camera shake index avoids the possibility of recording images merely for determining camera shake and then deleting them without using them for making the 3D data set.

An advantage of the method of the invention is that the analysis of the camera shake does not take place after scanning, i.e. after a 3D data set has been compiled, by looking for typical artifacts caused by camera shake, but is carried out prior to computation of the 3D data set. In this way, a 3D data set is only computed when only slight camera shake of the 3D camera is detected.

Another advantage is that the assessment of the degree of camera shake is not carried out subjectively according to the opinion of a user but takes place on the basis of objectively measurable criteria, so that the quality of the 3D data set is assured and errors due to human inobservance or inexperience are excluded.

Advantageously, the method further comprising: obtaining at least one scanning sequence, each formed by at least one respective group of the images of the object acquired while the at least one pattern is projected on the object, wherein the forming of the at least one comparative signal is performed for each scanning sequence, based on the group of images of that sequence, and the determining also includes determining whether the at least one camera shake index, determined with respect to each respective scanning sequence, satisfies predetermined acceptability criterion as a threshold value; and providing a 3D data set based on at least some of the scanning sequences for which the at least one camera shake index satisfies the predetermined acceptability criterion.

Advantageously, the at least one pattern has a brightness distribution with periodic phasing and a predetermined phase position relative to an illuminating ray, and the brightness distribution includes parallel stripes having a sinusoidal intensity gradient.

The brightness distribution can advantageously at least approximate a sinusoidal distribution, in order to make it possible to use common evaluation algorithms. According to the invention, a phase position of the at least one pattern in one of the at least two images is substantially identical to a phase position of the at least one pattern in another of the at least two images, or is shifted therefrom by a multiple of about half a period of the pattern.

The patterns of the at least two images used for computation of the camera shake index can have a relative phase position which is identical or is phase-shifted by a multiple of a half period of the pattern.

This makes for simple computation of the camera shake index. According to the invention, the individual images consist of a plurality of pixels showing levels of intensity or grayness, each pixel being associated with a pair of coordinates. The camera shake analyzing unit can form comparative signals from these images by division or subtraction, in which process two images showing a suitable relative phase position are used and the quotient or difference is formed for the levels of intensity of the images, pixel by pixel, such that the comparative signal again consists of a plurality of pixels.

The comparative signal can be formed by subtraction. To this end, the levels of intensity of a first image can be subtracted from the levels of intensity of a second image, pixel by pixel, so that the comparative signal shows a two-dimensional distribution. Advantageously, comparative signals can be determined by forming the difference between two images that are mutually out of phase by a half period of the pattern.

Since they are in mirror relationship to each other, subtraction leads in the case of sinusoidal signals to halving of the period with unchanged amplitude, if no camera shake has taken place. When a time-dependent camera shake of the 3D camera occurs, the comparative signal changes.

Instead of a difference, a quotient can be formed, in which case advantageously images showing the same phase position of the pattern are used.

Advantageously, division can be carried out, pixel by pixel, according to formula (1-q1/q2).

Advantageously, the forming includes forming at least two comparative signals based on at least three images of the object acquired by the camera while the at least one pattern is projected on the object, and the determining of the camera shake index is performed based on the at least two comparative signals.

Thus a camera shake index can be computed from at least two comparative signals, to which end at least three images are implemented.

This camera shake index is a reliable indication of camera shake of the 3D camera within a period of time elapsing between the exposures of the at least two images used for computation of the camera shake index.

Advantageously, merely a representative selection of pixels in the comparative signals can be used for computation of the camera shake index.

Thus the quality of the signal, i.e. of the camera shake index, is improved in, say, the center of the image.

Advantageously, the determining of the at least one camera shake index includes forming a signal variance of corresponding pixels of the at least two images.

Thus the computation of the camera shake index can be effected by forming the variance between the corresponding pixels of two images of identical phase position, in which case the squared deviations computed for computation of the variance are squares of the distances between corresponding pixels in the images being compared.

For this reason, the sum of the squared deviations, and thus also the variance, increases with increased camera shake so that the variance can be regarded as a suitable indicator of the degree of camera shake of the 3D camera.

Advantageously, the method further comprising: initiating an initiation request by actuating an initiator; within a predetermined time period, obtaining continuous scanning sequences formed by images of the object, acquired while the at least one pattern is projected on the object, wherein the forming of the at least one comparative signal is performed based on at least some of those images; and in response to a least value of the at least one camera shake index being determined, providing a 3D data set based on images in at least one of the scanning sequences obtained prior to identification of the least value.

Thus the user can execute an initiation request by actuating initiator such that, within a given subsequent time window, images can be continuously recorded both for scanning sequences and for computation of the camera shake index and these are temporarily stored in an image memory while the camera shake index is continuously computed. A moment of initiation at which there was a minimum camera shake can be selected and computation of a 3D image from the individual images in the preceding scanning sequence can be initiated.

In this way, the user can approximately fix the moment of exposure, while he tries to keep the 3D camera still following execution of the initiation request.

Advantageously, the specified time period can be up to two seconds. By this means the user will have adequate time to steady the 3D camera following actuation of the initiating means.

An advantageous development of the initiator consists in that the method further comprising: initiating an initiation request by actuating an initiator; after the initiation request, obtaining continuous scanning sequences formed by images of the object, acquired while the at least one pattern is projected on the object, wherein the forming of the at least one comparative signal is performed based on at some of those images; and in response to the at least one camera shake index falling below a threshold value, providing a 3D data set based on images in at least one of the scanning sequences.

Thus the user can execute an initiation request by actuating initiator such that, within a given subsequent time period, images both for a scanning sequence and for computation of the camera shake index are continuously recorded while the camera shake index is continuously computed and compared with the threshold value, and computation of at least one 3D data set from the images in the last scanning sequence is initiated when said index falls below of said threshold value and the 3D camera is thus in a relatively steady position.

In this way, the moment of scanning, i.e. the moment of exposure of the scanned data set used for computation of the 3D data set, can be determined by the user by steadying the 3D camera and allowing the camera shake index to fall short of a fixed threshold value. Such steadying typically rarely occurs accidentally. In this way it is possible to achieve considerable simplification of the scanning process, particularly in the case of a plurality of consecutive individual exposures for a 3D data set, since it is no longer necessary to monitor the results of the individual exposures.

Advantageously, the specified time period can here again be up to two seconds, in order to give the user adequate time to steady the 3D camera following actuation of the initiating means.

Advantageously, the threshold value is variable and increases, starting from a starting value, in a fixed time relationship until the measured camera shake index falls below the threshold value and thus computation of a 3D data set is initiated.

This can ensure that the measured camera shake index is under-run at at least one moment within the time period and that at least one computation of a 3D data set will be initiated.

Advantageously, the method further comprising: initiating an initiation request by actuating an initiator; and after the initiation request, obtaining continuous scanning sequences formed by images of the object, acquired while the at least one pattern is projected on the object, wherein the forming of the at least one comparative signal is performed based on at least some of those images, in response to each separate instance of the at least one camera shake index falling below a threshold, and without requiring further initiation of an initiating request, storing a respective 3D data set formed based on images in at least one of the scanning sequences obtained prior to the respective separate instance of the at least one camera shake index falling below the threshold, and combining at least some stored 3D data sets to form an overall 3D recording.

Thus after the actuation of the initiator by the user, each falling below of the threshold value during the entire time period of the scanning operation causes, without further initiation requests from the user, a 3D data set to be computed from the images in the preceding scanning sequence, which data set is then saved.

By this means, the user needs to initiate the scanning operation only once, and a number of 3D scans will occur whenever the user holds the 3D camera still and the camera shake index thus falls below of the threshold value.

Advantageously, at least five images with different phase positions of the pattern can be recorded, whereat at any one time the phase position is shifted by one quarter of the period of the pattern, that is to say, a first phase-related image with a phase position of 0° is recorded, then a second image with a phase position of 90°, a third image with a phase position of 180°, a fourth image with a phase position of 270° and finally a fifth image with a phase position of 360°. Thus the phase position of the final image corresponds to that of the image first recorded.

Advantageously, the first four images recorded form a scanning sequence, from which a 3D data set can be made by the phase shifting method.

A camera shake index can be advantageously computed by means of the camera shake analyzing unit from these five images recorded with the 3D camera, to which end a first comparative signal between the image of the second phase position and that of the fourth phase position is formed and a second comparative signal between the image of the first phase position and that of the third phase position is formed and a third comparative signal between the image of the third phase position and that of the fifth phase position is formed, in each case by subtraction, and the camera shake index is then computed by averaging a first signal variance between the first comparative signal and the second comparative signal and a second signal variance between the first comparative signal and the third comparative signal over a specific selection of a plurality of pixels, which camera shake index is implemented for the assessment of the degree of camera shake of the 3D camera during optical scanning.

When the 3D camera is in a position of rest, the first comparative signal between the second and fourth phase positions, the second comparative signal between the first and third phase positions, and the third comparative signal between the third and fifth phase positions have a sinusoidal shape with the same amplitude and half period as shown by the image signal of the pattern. Only when there is a time-dependent change in position of the 3D camera relative to the object being scanned is there a noticeable difference between the comparative signals. Consequently there is an increase in the first signal variance between the first comparative signal and the second comparative signal and also in the second signal variance between the first comparative signal and the third comparative signal, so that the average camera shake index rises. In this way the camera shake index is a reliable indicator of the camera shake of the 3D camera within a scanning sequence.

Advantageously, the camera shake analyzing unit can be implemented to compute a camera shake index from two images recorded with the 3D camera and having the same phase position, to which end a comparative signal can be formed by division according to formula (1-q1/q2) between the two images, and the camera shake index can be computed over a representative selection of a plurality of pixels of this comparative signal, which camera shake index is implemented for the assessment of the degree of camera shake of the 3D camera during the optical scan.

When the 3D camera is in a position of rest, the two images have the same phase position. Only when there is a time-dependent change in position of the 3D camera relative to the object being scanned do the two images differ from each other. Consequently, the quotient of the two images according to the aforementioned formula will deviate from zero and the camera shake index will rise. This means that the thus determined camera shake index is likewise a reliable indicator of the camera shake of the 3D camera within a scanning sequence.

For the purpose of visualizing the camera shake, a high-contrast image can be computed from at least two comparative signals that have been determined from at least three images of the pattern projected on the object and can be outputted. The oscillating camera movements typical of a handheld camera appear as a time-varying jitter.

This makes it possible for the user to observe the intensity of the camera shake in real time and to determine the moment of initiation correspondingly. The indicating device can be, say, a monitor.

Advantageously, the formula for computation of the high-contrast image can be $K = \sqrt{(\text{Im}^2 + \text{Re}^2)}$ wherein Im denotes the first comparative signal and Re the second comparative signal. Advantageously, the computed comparative signals can be temporarily stored in a data storage device and used for computation of another high-contrast image, in which the comparative signals from different scanning sequences are compared.

In order to obtain time-dependent visualization of the camera shake, two comparative signals determined from two images can be taken for continuous computation of a high-contrast image, which can be outputted, and, advantageously, use is always made of the last comparative signal to have been used and a subsequently determined comparative signal. The continuously outputted high-contrast images appear as a time-varying jitter.

The user can thus construe the degree of camera shake of the 3D camera in real time from the intensity of the jitter.

Advantageously, the high-contrast image is continuously computed and displayed over time and can be used by the user for choosing the moment of initiation for computation of the 3D data set from the individual images of a scanning sequence.

The high-contrast image thus allows for objective assessment of the degree of camera shake of the camera without previous experience, in order to recognize camera shake from the occurrence of artifacts in a viewfinder image known from the prior art.

Advantageously, at least four images can be used for computation of the high-contrast image, wherein the phase position of the pattern in each image is shifted by +90° relative to the preceding image so that the first phase position of the first image is 0°, the second phase position of the second image is 90°, the third phase position of the third image is 180° and the fourth phase position of the fourth image is 270°.

From the four images it is then possible to acquire two comparative signals by subtraction, a first comparative signal being formed from the images of a second phase position and a fourth phase position and a second comparative signal being formed between the images of a first phase position and a third phase position. Subsequently, for the purpose of visualization of the camera shake, a high-contrast image can be computed from the first comparative signal and the second comparative signal preceding the first comparative signal, which high-contrast image can be outputted on an indicating device.

Advantageously, the triangulation procedure is a double triangulation procedure, and wherein the method further comprises, for calculation of a 3D data set, forming a first scanning sequence at a first triangulation angle, and subsequently forming a second scanning sequence at a second triangulation angle, wherein the forming of the second scanning sequence is performed in response to the at least one camera shake index falling below a threshold value, and calculation of the 3D data set is performed based on images of the first scanning sequence acquired prior to the at least one camera shake index falling below the threshold value and based on images of the second scanning sequence.

Thus computation of an individual 3D data set being carried out by implementation of the first scanning sequence using a first triangulation angle and then of a second scanning sequence using a second triangulation angle.

The second scanning sequence can serve, for example, to enlarge the depth scanning range in that the monomode range is enlarged. This is known per se. In the context of the present invention, however, the second scanning sequence will only be initiated when the camera shake of the first scanning sequence is sufficiently small.

In the method of double triangulation, projection of the pattern onto the object is carried out at two different triangulation angles and recorded. In this way the uniquely assignable depth scanning range is increased distinctly. For this purpose, the first triangulation angle can be between 1° and 13° and the second triangulation angle between 1.5° and 15°. The unique depth scanning range can be between 20 mm and 25 mm.

Advantageously, according to the method of double triangulation, a second scanning sequence using a second triangulation angle can be recorded once the threshold was under-run, after which a 3D data set can be computed from the images in a first scanning sequence, prior to the point of falling short of the threshold value, and from images in the second scanning sequence.

A 3D data set can, according to the invention, be alternatively produced by using the light-section method, in which case a plurality of individual images of a plurality of patterns projected on the object are recorded to form a scanning sequence. From the positions of sub-patterns detected in the images of a scanning sequence geometric acquisition takes place and the 3D data set is computed.

According to the invention, at least one further image of the object showing one of the patterns is recorded, and this further image is used together with the image in the scanning sequence showing the corresponding pattern for determination of the camera shake index.

As another object of the invention, the invention relates to a portable dental 3D camera device having a triangulation angle, for optically scanning a threedimensional object to acquire a plurality of images of the object, the device comprising a camera shake analyzing unit, arranged to form at least one comparative signal by determining a quotient or difference of at least two images of the object acquired while a pattern is projected on the object, and to determine at least one camera shake index by averaging the at least one comparative signal.

Thus the dental 3D camera device is capable of recording at least two individual images of a pattern projected on the object. The device has a camera shake analyzing unit, and by means of the camera shake analyzing unit for determination of the camera shake of a handheld 3D camera at least one comparative signal can be acquired by division or subtraction of the at least two individual images, and a camera shake index can be computed from the at least one comparative signal.

This device of the invention is primarily suitable for the purpose of carrying out the method of the invention as defined in claim 1. According to the invention, the device comprising a image memory arranged to store a scanning sequence formed by at least two images of the object, acquired while the at least one pattern is projected on the object, the scanning sequence including at least one of the images used to form the at least one comparative signal for computation of the camera shake index; and a image processing unit arranged to provide at least one 3D data set based on at least some images of the scanning sequence.

Thus a scanning sequence consisting of at least two images recorded with the 3D camera is recorded and saved to the memory. Furthermore a 3D data set can be made from the images in the scanning sequence, whilst at least one of the images used for computation of the camera shake index can be part of the scanning sequence.

This has the advantage that a minimum of extra processing is necessary for ascertaining the degree of camera shake.

The device can have a pattern which can advantageously show a brightness distribution having periodic phases and a known phase relationship and a known phase position relative to an illuminating ray.

In particular, the brightness distribution can consist of parallel stripes showing a sinusoidal intensity gradient. Advantageously, the device further comprising an initiator operable to initiate an initiation signal, wherein in response thereto, and within a predetermined time period, the memory stores the scanning sequence and the image processing unit provides the at least one 3D data set, wherein the camera shake analyzing unit also is arranged to detect a least value of the at least one camera shake index, determined within the predetermined time period, and the processing unit is responsive to such a detection by providing the at least one 3D data set.

Thus the user can, by actuating the initiating means, initiate scanning and the computation of an individual 3D data set within a specified time period or alternatively scanning and the computation of a plurality of 3D data sets within the specified time window. The duration of the time period is stored in a memory in the camera shake analyzing unit.

The initiator can, for example, be a foot pedal or any desired actuating element.

Advantageously, the device can have an image processing unit, in which case the camera shake analyzing unit initiates computation of the 3D data set from the images in the selected scanning sequence in an image processing unit.

The image processing unit can be a data processing unit, such as a computer running special software for signal processing, or an electronic circuit.

Advantageously, the camera shake analyzing unit can comprise a memory in which a variable threshold value can be stored.

Advantageously, the device can comprise regulating means for changing the triangulation angle in order to make it possible to carry out optical scanning using the method of double triangulation involving a first scanning sequence at a first triangulation angle and a second scanning sequence at a second triangulation angle.

Such regulating means for changing the triangulation angle can be a mechanical flag for partial cutoff of the illuminating ray, some other electronically controlled optical diaphragm, or alternatively another light source, such that the regulating means causes the illuminating ray to come from a different direction and in this way alters the triangulation angle.

The computed comparative signals can be stored in a data storage device present in the camera shake analyzing unit.

Advantageously, the device can have indicating means, which can be in the form of a monitor, and a high-contrast image of at least two comparative signals can be computed for visualization of the camera shake, which comparative signals can be acquired, by subtraction or division, from at least three images of the pattern projected on the object. The high-contrast image can be indicated by the indicating means as a simultaneously displayed number of measured values depicting pixel intensities, the camera shake appearing in the form of a time-varying jitter. The camera shake analyzing unit can be designed such that it is suitable for computation of the high-contrast image.

The camera shake analyzing unit can be installed in the 3D camera or remote therefrom in data processing equipment connected to the 3D camera by a cable or wireless. The camera shake analyzing unit can be a computer running software specially adapted for signal processing, or alternatively an electronic circuit adapted to process incoming signals in a prescribed manner and to emit an output signal. This camera shake analyzing unit computes, for example, the camera shake index in said manner by comparing the incoming signals from the individual images with each other by differential analysis.

Brief description of the drawings

An exemplary embodiment of the invention is shown in the drawings, in which

Fig. 1    is a diagram for clarification of the method of the invention showing an initiator and an automatic initiation and

Fig. 2    is a graphical representation to clarify the computation of the camera shake index.

Exemplary embodiments

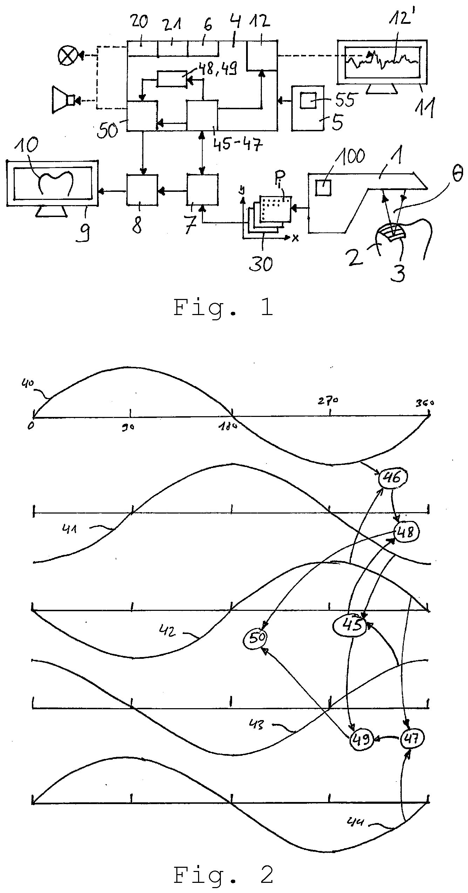

Fig. 1 is a diagram for clarification of the method of the invention and the device of the invention as exemplified by the phase shifting method. A dental 3D camera 1 is used in order to scan a three-dimensional object 2, namely a tooth, using a triangulation method. A viewfinder image, not shown but known from the prior art, makes it possible to position the 3D camera 1 over the object 2. The 3D camera 1 can then record five individual images 30 of the pattern 3 projected on the object 2, which pattern 3 can consist of parallel stripes having a sinusoidal brightness distribution, and the images 30 can each contain a plurality of pixels $P_i$ having the coordinates $(x_i, y_i)$. For each image 30 the pattern 3 can be shifted by a quarter of a period, i.e. a phase of 90°, the first four of the five images 30 forming a scanning sequence, from which a 3D data set 10 of the object 2 can be computed.

The recorded images 30 can be transmitted by the 3D camera 1 to an image memory 7 and can be evaluated by a camera shake analyzing unit 4 in which the data pertaining to the images 30 are implemented for computation of a camera shake index 50. To this end, comparative signals 45, 46, 47 can be determined by subtraction or division between individual images 30 at corresponding phase positions 40, 41, 42, 43, 44 and can be stored in a data storage device 21. The camera shake index 50 can be determined by averaging or subtracting these comparative signals 45, 46, 47 and by carrying out further averaging over a relevant selection of a plurality of pixels $P_i$. The way in which the computation is carried out is explained in greater detail with reference to Fig. 2.

When the user actuates the initiator 5, which in the present embodiment can be a footswitch, an initiation request is sent to the camera shake analyzing unit 4. Within a specified time period of, say, 2 seconds, the duration of which is stored in a data storage device 6, images 30 are recorded and placed in an image memory 7, and the camera shake index 50 is computed.

It is then possible to determine a moment of initiation with the lowest camera shake index 50 within the time period and the four images 30 of the scanning sequence preceding said moment of initiation can be fetched from the image memory 7 and passed on to an image processing unit 8. A 3D data set 10 can be computed in the image processing unit 8 from the four individual images 30 by the phase shifting method known per se.

The 3D data set 10 contains the three-dimensional data pertaining to the recorded object 2 and is displayed on an indicating device 9 as a three-dimensional model of the scanned object 2, namely a tooth.

In another embodiment, the computed camera shake index 50 can, within the time period following actuation of the initiator 5, be compared with a variable threshold value stored in the memory 20, and in the case of a falling short of the threshold value, the computation of the 3D data set 10 of images 30 from the preceding scanning sequence is initiated.

The camera shake analyzing unit 4 can in addition compute a high-contrast image 12 from the first comparative signal 45 and either from the second, preceding comparative signal 46 or from the third, succeeding comparative signal 47 of the individual images 30 and can be shown by means of an indicating device 11 as a time-varying jitter 12'. The high-contrast image 12 reproduces the camera shake of the 3D camera in real time. The user can thus directly watch the fluctuations of the camera shake with time. The user can utilize this high-contrast image 12, for example, in order to assess the degree of camera shake and, for example, to initiate computation of the 3D data set 10 from images 30 in the preceding scanning sequence by again actuating the initiator 5 at a moment of low camera shake as indicated by the high-contrast image 12.

The data storage device is advantageously large enough to store the data of all comparative signals computed during the entire optical scanning operation.

In addition, the camera shake index can be outputted, for example, in the form of a lamp signal or an acoustic signal. For example, the camera shake index can be continuously compared with the threshold value and, depending on whether it falls short of or exceeds the threshold value, the lamp will emit light or not.

Fig. 2 is a diagrammatic representation for clarification of the computation of the camera shake index 50 and the high-contrast image 12 as exemplified by the phase shifting method. The first phase position 40 of the first image 30 is 0°, the second phase position 41 of the second image 30 is 90°, the third phase position 42 of the third image 30 is 180°, the fourth phase position 43 of the fourth image 30 is 270°, and the fifth phase position of the fifth image 30 is 360°. A first comparative signal 45 is formed by the addition of the phasing 41 of the second image 30 to the phasing 43 of the fourth image 30. A second comparative signal 46 is formed by the addition of the phasing 40 of the first image 30 to the phasing 42 of the third image 30. A third comparative signal 47 is formed by the addition of the phasing 42 of the third image 30 to the phasing 44 of the fifth image 30. A first signal variance 48 is formed from the first comparative signal 45 and the second comparative signal 46, a second signal variance is formed from the first comparative signal 45 and the third comparative signal 47, and the camera shake index 50 is computed by averaging over a relevant range of pixels $P_i$.

List of reference numerals or characters

| | |
|---|---|
| 1 | dental 3D camera |
| 2 | object |
| 3 | pattern |
| 4 | camera shake analyzing unit |
| 5 | initiator |
| 6 | data storage device for the duration of the scanning time period |
| 7 | image memory |
| 8 | image processing unit |
| 9 | indicating device for indication of the 3D data set |
| 10 | 3D data set |
| 11 | indicating device for indication of the high-contrast image |
| 12 | high-contrast image |
| 20 | memory for the threshold value |
| 21 | data storage device for the comparative signals |
| 30 | images having pixels $P_i$ and having levels of intensity or grayness $q_i$ |
| 40 | first phase position |
| 41 | second phase position |
| 42 | third phase position |
| 43 | fourth phase position |
| 44 | fifth phase position |
| 45 | first comparative signal |
| 46 | second comparative signal |
| 47 | third comparative signal |
| 48 | first signal variance |
| 49 | second signal variance |
| 50 | camera shake index |
| 55 | switch |
| 100 | regulating means |

**Claims**

1. A method for optically scanning a threedimensional object (2) with a dental 3D camera (1) that operates in accordance with a triangulation procedure to acquire a plurality of images (30) of the object (2), the method comprising:

   forming at least one comparative signal (45, 46, 47) based on at least two images (30) of the object (2) acquired by the camera (1) while at least one pattern (3) is projected on the object (2), **characterized in that** at least one camera shake index (50) based on the at least one comparative signal (45, 46, 47) is determined, wherein the forming of the at least one comparative signal is performed based on at least one of the images for providing a 3D data set, and wherein a phase position (40, 41, 42, 43, 44) of the at least one pattern (3) in one of the at least two images (30) is substantially identical to a phase position (40, 41, 42, 43, 44) of the at least one pattern (3) in another of the at least two images (30), or is shifted therefrom by a multiple of about half a period of the pattern (3) wherein the images (30) include a plurality of pixels (Pi) having coordinates ($x_i$, $y_i$) showing levels of intensity or grayness (qi), the at least one comparative signal (45, 46, 47) comprises a plurality of pixels (Pi), and the forming of the at least one comparative signal (45, 46, 47) includes dividing or subtracting two images (30) of the pattern (3) of said phase positions (40, 41, 42, 43, 44) to obtain a quotient or difference, respectively, formed for levels of intensity or grayness (qi) of those images (30) on a pixel-by-pixel basis.

2. The method as defined in claim 1, further comprising:

obtaining at least one scanning sequence, each formed by at least one respective group of the images (30) of the object (2) acquired while the at least one pattern (3) is projected on the object (2), wherein the forming of the at least one comparative signal (45, 46, 47) is performed for each scanning sequence, based on the group of images (30) of that sequence, and the determining also includes determining whether the at least one camera shake index (50), determined with respect to each respective scanning sequence, satisfies predetermined acceptability criterion as a threshold value;
and providing a 3D data set (10) based on at least some of the scanning sequences for which the at least one camera shake index (50) satisfies the predetermined acceptability criterion.

3. The method as defined in claim 1 or claim 2, wherein the at least one pattern (3) has a brightness distribution with periodic phasing and a predetermined phase position (40, 41, 42, 43, 44) relative to an illuminating ray, and the brightness distribution includes parallel stripes having a sinusoidal intensity gradient.

4. The method as defined in any one of claims 1 to 3, wherein the forming includes forming at least two comparative signals (45, 46, 47) based on at least three images (30) of the object (2) acquired by the camera while the at least one pattern (3) is projected on the object (2), and the determining of the camera shake index (50) is performed based on the at least two comparative signals (45, 46, 47).

5. The method as defined in any one of claims 1 to 3, wherein the determining of the at least one camera shake index (50) includes forming a signal variance (48, 49) of corresponding pixels (Pi) of the at least two images (30).

6. The method as defined in any one of claims 1 to 5, further comprising: initiating an initiation request by actuating an initiator (5, 55); within a predetermined time period, obtaining continuous scanning sequences formed by images (30) of the object (2), acquired while the at least one pattern (3) is projected on the object (2), wherein the forming of the at least one comparative signal (45, 46, 47) is performed based on at least some of those images (30); and in response to a least value of the at least one camera shake index (50) being determined, providing a 3D data set (10) based on images (30) in at least one of the scanning sequences obtained prior to identification of the least value.

7. The method as defined in any one of claims 1 to 6, further comprising: initiating an initiation request by actuating an initiator (5, 55); after the initiation request, obtaining continuous scanning sequences formed by images (30) of the object (2), acquired while the at least one pattern (3) is projected on the object (2), wherein the forming of the at least one comparative signal (45, 46, 47) is performed based on at some of those images (30); and in response to the at least one camera shake index (50) falling below a threshold value, providing a 3D data set (10) based on images (30) in at least one of the scanning sequences.

8. The method as defined claims 7, wherein the threshold value is variable and increases, starting from a starting value, in a fixed time relationship until the measured camera shake index (50) falls below the threshold value and thus computation of a 3D data set (10) is initiated.

9. The method as defined in any one of claims 1 to 8, further comprising: initiating an initiation request by actuating an initiator (5, 55); and after the initiation request, obtaining continuous scanning sequences formed by images (30) of the object (2), acquired while the at least one pattern (3) is projected on the object (2), wherein the forming of the at least one comparative signal (45, 46, 47) is performed based on at least some of those images (30), in response to each separate instance of the at least one camera shake index (50) falling below a threshold, and without requiring further initiation of an initiating request, storing a respective 3D data set (10) formed based on images (30) in at least one of the scanning sequences obtained prior to the respective separate instance of the at least one camera shake index (50) falling below the threshold, and combining at least some stored 3D data sets (10) to form an overall 3D recording.

10. The method as defined in any one of claims 1 to 9, wherein the triangulation procedure is a double triangulation procedure, and wherein the method further comprises, for calculation of a 3D data set, forming a first scanning sequence at a first triangulation angle ($\theta1$), and subsequently forming a second scanning sequence at a second triangulation angle ($\theta2$), wherein the forming of the second scanning sequence is performed in response to the at least one camera shake index (50) falling below a threshold value, and calculation of the 3D data set (10) is performed based on images (30) of the first scanning sequence acquired prior to the at least one camera shake index (50) falling below the threshold value and based on images (30) of the second scanning sequence.

11. A portable dental 3D camera device having a triangulation angle ($\theta$), for optically scanning a threedimensional object (2) to acquire a plurality of images (30) of the object (2), the device comprising a camera shake analyzing unit (4), arranged to form at least one comparative signal (45, 46, 47) by determining a quotient or difference of at least two images (30) of the object (2) acquired while a pattern (3) is projected on the object (2), and to determine at least one camera shake index (50) by averaging the at least one comparative signal (45, 46, 47), further comprising: a image memory (7) arranged to store a scanning sequence formed by at least two images (30) of the object (2), acquired while the at least one pattern (3) is projected on the object (2), the scanning sequence including at least one of the images (30) used to form the at least one comparative signal (45, 46, 47) for computation of the camera shake index (50); and a image processing unit (8) arranged to provide at least one 3D data set (10) based on at least some images (30) of the scanning sequence.

12. The device as defined in claim 11, further comprising an initiator (5, 55) operable to initiate an initiation signal, wherein in response thereto, and within a predetermined time period, the memory stores the scanning sequence and the image processing unit (8) provides the at least one 3D data set (10), wherein the camera shake analyzing unit (4) also is arranged to detect a least value of the at least one camera shake index (50), determined within the predetermined time period, and the processing unit (8) is responsive to such a detection by providing the at least one 3D data set (10).

**Patentansprüche**

1. Verfahren zum optischen Abtasten eines dreidimensionalen Gegenstandes (2) mit einer 3D-Dentalkamera (1), die nach einem Triangulationsverfahren arbeitet, um eine Vielzahl an Bildern (30) eines Gegenstandes (2) zu erfassen, das Verfahren Folgendes umfassend:

Bilden mindestens eines Vergleichssignals (45, 46, 47) basierend auf mindestens zwei Bildern (30) des Gegenstandes (2), die von der Kamera (1) erfasst wurden, während mindestens ein Muster (3) auf den Gegenstand (2) projiziert wird, **dadurch gekennzeichnet, dass** mindestens ein auf dem mindestens einen Vergleichssignal (45, 46, 47) basierender Kameraverwacklungsindex (50) bestimmt wird , worin das Bilden mindestens eines Vergleichssignals basierend auf mindestens einem der Bilder zur Bereitstellung eines 3D-Datensatzes ausgeführt wird, und

worin eine Phasenlage (40, 41, 42, 43, 44) mindestens eines Musters (3) in einem der mindestens zwei Bilder (30) im Wesentlichen mit einer Phasenlage (40, 41, 42, 43, 44) des mindestens einen Musters (3) in einem anderen der mindestens zwei Bilder (30) identisch oder davon durch ein Vielfaches etwa einer halben Periode des Musters (3) verschoben ist, worin die Bilder (30) eine Vielzahl von Pixeln (Pi) mit Koordinaten ($x_i$, $y_i$) enthalten, die Intensitätsstufen oder Graustufen (qi) anzeigen, wobei das mindestens eine Vergleichssignal (45, 46, 47) eine Vielzahl von Pixeln (Pi) umfasst und das Bilden des mindestens einen Vergleichssignals (45, 46, 47) das Dividieren oder Subtrahieren von zwei Bildern (30) des Musters (3) der genannten Phasenlagen (40, 41, 42, 43, 44) beinhaltet, um Pixel für Pixel jeweils einen Quotienten oder eine Differenz zu erhalten, der bzw. die jeweils für Intensitäts- oder Graustufen (qi) dieser Bilder (30) gebildet wird.

2. Verfahren nach Anspruch 1, des Weiteren Folgendes umfassend:

Erhalten mindestens einer Abtastfolge, jeweils gebildet durch mindestens eine entsprechende Gruppe der Bilder (30) des Gegenstandes (2), die erfasst wurden, während das mindestens eine Muster (3) auf den Gegenstand (2) projiziert wird, worin das Bilden des mindestens einen Vergleichssignals (45, 46, 47) für jede Abtastfolge basierend auf der Gruppe von Bildern (30) dieser Folge durchgeführt wird, und wobei das Bestimmen außerdem beinhaltet, dass bestimmt wird, ob der mindestens eine Kameraverwacklungsindex (50), der in Bezug auf jede entsprechende Abtastfolge bestimmt wurde, ein vorbestimmtes Annahmekriterium als ein Schwellenwert erfüllt; und Bereitstellen eines 3D-Datensatzes (10), der auf mindestens einigen der Abtastfolgen basiert, für die der mindestens eine Kameraverwacklungsindex (50) das vorbestimmte Annahmekriterium erfüllt.

3. Verfahren wie in Anspruch 1 oder Anspruch 2 definiert, worin das mindestens eine Muster (3) eine Helligkeitsverteilung mit periodischen Phasen und eine vorbestimmte Phasenlage (40, 41, 42, 43, 44) in Bezug auf einen Beleuchtungsstrahl aufweist und die Helligkeitsverteilung parallele Streifen beinhaltet, die einen sinusförmigen Intensitätsgradienten aufweisen.

**4.** Verfahren wie in einem der Ansprüche 1 bis 3 definiert, worin das Bilden das Bilden von mindestens zwei Vergleichssignalen (45, 46, 47) beinhaltet, die auf mindestens drei Bildern (30) des Gegenstandes (2) basieren, die von der Kamera erfasst wurden, während das mindestens eine Muster (3) auf den Gegenstand (2) projiziert wird, und wobei das Bestimmen des Kameraverwacklungsindex (50) basierend auf den mindestens zwei Vergleichssignalen (45, 46, 47) ausgeführt wird.

**5.** Verfahren wie in einem der Ansprüche 1 bis 3 definiert, worin das Bestimmen des mindestens einen Kameraverwacklungsindex (50) das Bilden einer Signalvarianz (48, 49) der entsprechenden Pixel (Pi) der mindestens zwei Bilder (30) beinhaltet.

**6.** Verfahren wie in einem der Ansprüche 1 bis 5 definiert, des Weiteren Folgendes umfassend: Auslösen einer Auslöseanforderung durch Betätigen eines Auslösers (5, 55); innerhalb eines vorbestimmten Zeitraums, Erhalten kontinuierlicher Abtastfolgen, die durch Bilder (30) des Gegenstandes (2) gebildet wurden, die erfasst wurden, während das mindestens eine Muster (3) auf den Gegenstand (2) projiziert wird, worin das Bilden des mindestens einen Vergleichssignals (45, 46, 47) basierend auf mindestens einigen dieser Bilder (30) durchgeführt wird; und Bereitstellen eines 3D-Datensatzes (10), der auf Bildern (30) in mindestens einer der vor der Identifizierung des geringsten Wertes erhaltenen Abtastfolgen basiert, als Reaktion auf einen geringsten Wert des mindestens einen Kameraverwacklungsindex (50), der bestimmt wird.

**7.** Verfahren wie in einem der Ansprüche 1 bis 6 definiert, des Weiteren Folgendes umfassend: Auslösen einer Auslöseanforderung durch Betätigen eines Auslösers (5, 55); nach der Auslöseanforderung Erhalten kontinuierlicher Abtastfolgen, die durch Bilder (30) des Gegenstandes (2) gebildet wurden, die erfasst wurden, während das mindestens eine Muster (3) auf den Gegenstand (2) projiziert wird, worin das Bilden des mindestens einen Vergleichssignals (45, 46, 47) basierend auf einigen dieser Bilder (30) durchgeführt wird; und, Bereitstellen eines auf Bildern (30) in mindestens einer der Abtastfolgen basierenden 3D-Datensatzes (10) als Reaktion auf den mindestens einen Kameraverwacklungsindex (50), der unter einen Schwellenwert fällt.

**8.** Verfahren wie in Anspruch 7 definiert, worin der Schwellenwert veränderlich ist und ausgehend von einem Anfangswert in einer festen Zeitbeziehung steigt, bis der gemessene Kameraverwacklungsindex (50) unter den Schwellenwert fällt und somit die Berechnung eines 3D-Datensatzes (10) ausgelöst wird.

**9.** Verfahren wie in einem der Ansprüche 1 bis 8 definiert, des Weiteren umfassend: Auslösen einer Auslöseanforderung durch Betätigen eines Auslösers (5, 55); und, nach der Auslöseanforderung, Erhalten kontinuierlicher Abtastfolgen, die durch Bilder (30) des Gegenstandes (2) gebildet werden, die erfasst werden, während das mindestens eine Muster (3) auf den Gegenstand (2) projiziert wird, worin das Bilden des mindestens einen Vergleichssignals (45, 46, 47) basierend auf mindestens einigen dieser Bilder (30) als Reaktion auf jede einzelne Instanz des mindestens einen Kameraverwacklungsindex (50) durchgeführt wird, der unter einen Schwellwert fällt, und ohne dass eine weitere Auslösung einer Auslöseanforderung erforderlich ist, Speichern eines entsprechenden 3D-Datensatzes (10), der basierend auf Bildern (30) in mindestens einer der Abtastfolgen gebildet wird, die erhalten wurden, bevor die entsprechende einzelne Instanz des mindestens einen Kameraverwacklungsindex (50) unter den Schwellenwert fällt, und Kombinieren mindestens einiger gespeicherter 3D-Datensätze (10), um eine 3D-Gesamtaufzeichnung zu bilden.

**10.** Verfahren wie in einem der Ansprüche 1 bis 9 definiert, worin das Triangulationsverfahren ein Verfahren doppelter Triangulation ist, und worin das Verfahren des Weiteren zur Berechnung eines 3D-Datensatzes das Bilden einer ersten Abtastfolge in einem ersten Triangulationswinkel ($\theta1$) und anschließendes Bilden einer zweiten Abtastfolge in einem zweiten Triangulationswinkel ($\theta2$) umfasst, worin das Bilden der zweiten Abtastfolge als Reaktion darauf durchgeführt wird, dass der mindestens eine Kameraverwacklungsindex (50) unter einen Schwellenwert fällt, und das Berechnen des 3D-Datensatzes (10) basierend auf Bildern (30) der ersten Abtastfolge, die erfasst wurden, bevor der mindestens eine Kameraverwacklungsindex (50) unter den Schwellenwert fällt, und basierend auf Bildern (30) der zweiten Abtastfolge durchgeführt wird.

**11.** Tragbare 3D-Dentalkameravorrichtung mit einem Triangulationswinkel ($\theta$) zum optischen Abtasten eines dreidimensionalen Gegenstandes (2) zum Erfassen einer Vielzahl von Bildern (30) des Gegenstandes (2), wobei die Vorrichtung eine Kameraverwacklungsauswertungseinheit (4) umfasst, die ausgeführt ist, um mindestens ein Vergleichssignal (45, 46, 47) durch Bestimmung eines Quotienten oder einer Differenz von mindestens zwei Bildern (30) des Gegenstandes (2) zu bilden, die erfasst wurden, während ein Muster (3) auf den Gegenstand (2) projiziert wird, und um mindestens einen Kameraverwacklungsindex (50) durch Ermittlung des mindestens einen Vergleichssignals (45, 46, 47) zu bestimmen, des Weiteren Folgendes umfassend: ein Bildspeicher (7), der zur Speicherung einer Abtastfolge eingerichtet ist, die durch mindestens zwei Bilder (30) des Gegenstandes (2) gebildet wird, die erfasst wurden, während das mindestens eine Muster (3) auf den Gegenstand (2) projiziert wird, wobei die Abtastfolge mindestens eines des zur Bildung des mindestens einen Vergleichssignals (45, 46, 47) verwendeten Bildes (30) zur Berechnung des Kameraverwacklungsindex (50) beinhaltet; und eine Bildverarbeitungseinheit (8), die so ausgebildet ist, dass sie basierend auf mindestens einigen Bildern (30) der Abtastfolge mindestens einen 3D-Datensatz (10) bereitstellt.

**12.** Vorrichtung wie in Anspruch 11 definiert, des Weiteren umfassend einen Auslöser (5, 55), der betätigt werden kann, um ein Auslösesignal auszulösen, worin der Speicher als Reaktion darauf und innerhalb eines vorbestimmten Zeitraums die Abtastfolge speichert und die Bildverarbeitungseinheit (8) den mindestens einen 3D-Datensatz (10) bereitstellt, worin die Kameraverwacklungsauswertungseinheit (4) weiterhin so ausgebildet ist, dass sie einen geringsten Wert des mindestens einen Kameraverwacklungsindex (50) erfasst, der innerhalb des vorbestimmten Zeitraums bestimmt wurde, und die Verarbeitungseinheit (8) auf eine solche Erfassung durch Bereitstellen des mindestens einen 3D-Datensatzes (10) anspricht.

## Revendications

**1.** Procédé permettant d'effectuer un balayage optique sur un objet (2) avec une caméra 3D (1) qui fonctionne conformément à une procédure de triangulation pour acquérir une pluralité d'images (30) de l'objet (2), le procédé comprenant :

la formation d'au moins un signal comparatif (45, 46, 47) sur la base d'au moins deux images (30) de l'objet (2) acquises par la caméra (1) pendant qu'au moins un motif (3) est projeté sur l'objet (2), **caractérisé en ce qu'**au moins un indice de vibration de caméra (50) basé sur l'au moins un signal comparatif (45, 46, 47) est déterminé, la formation de l'au moins un signal comparatif étant réalisée sur la base d'au moins l'une des images pour fournir un jeu de données 3D, et une position de phase (40, 41, 42, 43, 44) de l'au moins un motif (3) dans l'une des au moins deux images (30) étant sensiblement identique à une position de phase (40, 41, 42, 43, 44) de l'au moins un motif (3) dans une autre des au moins deux images (30), ou étant décalée par rapport à celle-ci d'un multiple d'environ la moitié d'une période du motif (3), les images (30) comprenant une pluralité de pixels (Pi) ayant des coordonnées $(x_i, y_i)$ montrant des taux d'intensité ou de gris (qi), l'au moins un signal comparatif (45, 46, 47) comprenant une pluralité de pixels (Pi), et la formation de l'au moins signal comparatif (45, 46, 47) comprenant la division ou la soustraction de deux images (30) du motif (3) desdites positions de phase (40, 41, 42, 43, 44) pour obtenir un quotient ou une différence, respectivement, formé pour des taux d'intensité ou de gris (qi) de ces images (30) sur une base par pixel.

**2.** Procédé tel que défini dans la revendication 1, comprenant en outre :

l'obtention d'au moins une séquence de balayage, chacune formée par au moins un groupe respectif des images (30) de l'objet (2) acquises pendant que l'au moins un motif (3) est projeté sur l'objet (2), la formation de l'au moins un signal comparatif (45, 46, 47) étant réalisée pour chaque séquence de balayage, sur la base du groupe d'images (30) de cette séquence, et la détermination comprenant également la détermination du fait qu'au moins un indice de vibration de caméra (50), défini vis-à-vis de chaque séquence de balayage, satisfait ou non un critère d'admissibilité prédéfini en tant que valeur seuil ;
et la fourniture d'un jeu de données 3D (10) sur la base d'au moins certaines des séquences de balayage pour lesquelles l'au moins un indice de vibration de caméra (50) satisfait le critère d'admissibilité prédéfini.

**3.** Procédé tel que défini dans la revendication 1 ou la revendication 2, dans lequel l'au moins un motif (3) présente une distribution de luminosité avec un phasage périodique et une position de phase (40, 41, 42, 43, 44) prédéfinie par rapport à un rayon d'éclairage, et la distribution de luminosité comprend des bandes parallèles ayant un gradient d'intensité sinusoïdal.

**4.** Procédé tel que défini dans l'une quelconque des revendications 1 à 3, dans lequel la formation comprend la formation d'au moins deux signaux comparatifs (45, 46, 47) sur la base d'au moins trois images (30) de l'objet (2) acquises par la caméra pendant que l'au moins un motif (3) est projeté sur l'objet (2), et la détermination de l'indice de vibration de caméra (50) est réalisée sur la base des au moins deux signaux comparatifs (45, 46, 47).

**5.** Procédé tel que défini dans l'une quelconque des revendications 1 à 3, dans lequel la détermination de l'au moins un indice de vibration de caméra (50) comprend la formation d'une variance de signal (48, 49) de pixels (Pi) correspondants des au moins deux images (30).

6. Procédé tel que défini dans l'une quelconque des revendications 1 à 5, comprenant en outre : l'initiation d'une requête d'initiation par l'actionnement d'un initiateur (5, 55) ; dans un délai prédéterminé, l'obtention de séquences de balayage continues formées par des images (30) de l'objet (2), acquises pendant que l'au moins un motif (3) est projeté sur l'objet (2), la formation de l'au moins un signal comparatif (45, 46, 47) étant réalisée sur la base certaines de ces images (30) ; et en réponse à la détermination d'une moindre valeur de l'au moins un indice de vibration de caméra (50), la fourniture d'un jeu de données 3D (10) sur la base d'images (30) dans au moins l'une des séquences obtenues avant l'identification de la moindre valeur.

7. Procédé tel que défini dans l'une quelconque des revendications 1 à 6, comprenant en outre : l'initiation d'une requête d'initiation par l'actionnement d'un initiateur (5, 55) ; après la requête d'initiation, l'obtention de séquences de balayage continues formées par des images (30) de l'objet (2) acquises pendant que l'au moins un motif (3) est projeté sur l'objet (2), la formation de l'au moins un signal comparatif (45, 46, 47) étant réalisée sur la base d'au moins certaines de ces images (30) ; et en réponse à la chute de l'au moins un indice de vibration de caméra (50) au-dessous d'une valeur seuil, la fourniture d'un jeu de données 3D (10) sur la base d'images (30) dans au moins l'une des séquences de balayage.

8. Procédé tel que défini dans la revendication 7, dans lequel la valeur seuil est variable et augmente, en partant d'une valeur de départ, dans une relation à durée fixe jusqu'à la chute de l'indice de vibration de caméra (50) mesuré sous la valeur seuil et ainsi l'initiation du calcul d'un jeu de données 3D (10).

9. Procédé tel que défini dans l'une quelconque des revendications 1 à 8, comprenant en outre : l'initiation d'une requête d'initiation par l'actionnement d'un initiateur (5, 55) ; et après la requête d'initiation, l'obtention de séquences de balayage continues formées par des images (30) de l'objet (2), acquises pendant que l'au moins un motif (3) est projeté sur l'objet (2), la formation de l'au moins un signal comparatif (45, 46, 47) étant réalisée sur au moins certaines de ces images (30), en réponse à chaque cas séparé de chute de l'au moins un indice de vibration de caméra (50) sous un seuil, et sans nécessiter d'initiation supplémentaire d'une requête d'initiation, le stockage d'un jeu de données 3D (10) respectif formé sur la base d'images (30) dans au moins l'une des séquences de balayage obtenues avant le cas séparé respectif de la chute de l'au moins un indice de vibration de caméra (50) sous le seuil, et la combinaison d'au moins certains jeux de données 3D (10) stockés pour former un enregistrement 3D global.

10. Procédé tel que défini dans l'une quelconque des revendications 1 à 9 , dans lequel la procédure de triangulation est une procédure de double triangulation, le procédé comprenant en outre, pour le calcul d'un jeu de données 3D, la formation d'une première séquence de balayage selon un premier angle de triangulation ($\theta$1), et la formation ultérieure d'une deuxième séquence de balayage selon un deuxième angle de triangulation ($\theta$2), la formation de la deuxième séquence de balayage étant réalisée en réponse à la chute de l'au moins un indice de vibration de caméra (50) sous une valeur seuil, et le calcul du jeu de données 3D (10) étant réalisé sur la base d'images (30) de la première séquence de balayage acquises avant la chute de l'au moins un indice de vibration de caméra (50) sous la valeur seuil et sur la base d'images (30) de la deuxième séquence de balayage.

11. Dispositif de caméra 3D dentaire portable ayant un angle de triangulation ($\theta$), permettant d'effectuer un balayage optique d'un objet tridimensionnel (2) pour acquérir une pluralité d'images (30) de l'objet (2), le dispositif comprenant une unité d'analyse de vibration de caméra (4), agencée pour former au moins un signal comparatif (45, 46, 47) par la détermination d'un quotient ou d'une différence d'au moins deux images (30) de l'objet (2) acquises pendant qu'un motif (3) est projeté sur l'objet (2), et déterminer au moins un indice de vibration de caméra (50) par l'établissement d'une moyenne de l'au moins un signal comparatif (45, 46, 47), comprenant en outre : une mémoire d'image (7) agencée pour stocker une séquence de balayage formée par au moins deux images (30) de l'objet (2), acquises pendant que l'au moins un motif (3) est projeté sur l'objet (2), la séquence de balayage comprenant au moins l'une des images (30) utilisées pour former l'au moins un signal comparatif (45, 46, 47) pour le calcul de l'indice de vibration de caméra (50) ; et une unité de traitement d'image (8) agencée pour fournir au moins un jeu de données 3D (10) sur la base d'au moins certaines images (30) de la séquence de balayage.

12. Dispositif tel que défini dans la revendication 11, comprenant en outre un initiateur (5, 55) pouvant fonctionner pour initier un signal d'initiation, la mémoire, en réponse à celui-ci et dans une durée prédéfinie, stockant la séquence de balayage et l'unité de traitement d'image (8) fournissant l'au moins un jeu de données 3D (10), l'unité d'analyse de vibration de caméra (4) étant également agencée pour détecter une moindre valeur de l'au moins un indice de vibration de caméra (50), défini dans la durée prédéfinie, et l'unité de traitement (8) répondant à une telle détection en fournissant l'au moins un jeu de données 3D (10).

Fig. 1

Fig. 2

**EP 2 198 780 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102005020240 A1
- EP 0250993 B1
- DE 19829278
- JP 2008190962 A